# EUROPEAN PATENT SPECIFICATION

(11) **EP 0 716 303 B1**
(45) Date of publication and mention of the grant of the patent: **02.02.2000**
(21) Application number: 95118207.0
(22) Date of filing: 20.11.1995
(51) Int. Cl.: G01N 33/34, G01N 21/35, G01N 27/22

(54) **A system related apparatus for the calibration of a sensor for moisture measurement on sheet, tape or film materials**
Einrichtung zum Eichen eines Sensors zur Bestimmung der Feuchtigkeit von Blatt-, Band- oder Folien-Materialien
Appareil de calibration d'un capteur pour la mesure de la teneur en eau de matériaux en feuilles, en bandes ou en pellicules

(30) Priority: 06.12.1994 IT MI942466
(43) Date of publication of application: 12.06.1996
(73) Proprietor: ELECTRONIC SYSTEMS S.P.A., I-28015 Momo (No) (IT)
(72) Inventor: Masotti, Alessandro, I-28040 Lesa (NO) (IT)
(74) Representative: Savi, Alberto

(56) References cited:
- EP-A- 0 453 797
- WO-A-95/34076
- PATENT ABSTRACTS OF JAPAN vol. 008, no. 109 (P-275), 22 May 1984 & JP-A-59 017141 (KETSUTO KAGAKU KENKYUSHO:KK), 28 January 1984,
- PATENT ABSTRACTS OF JAPAN vol. 94, no. 011 & JP-A-06 324544 (FUJI XEROX CO LTD), 25 November 1994,
- PATENT ABSTRACTS OF JAPAN vol. 013, no. 229 (M-831), 26 May 1989 & JP-A-01 041740 (NATL HOUSE IND CO LTD), 14 February 1989,
- PATENT ABSTRACTS OF JAPAN vol. 018, no. 611 (M-1708), 21 November 1994 & JP-A-06 229658 (SANYO ELECTRIC CO LTD), 19 August 1994,
- PATENT ABSTRACTS OF JAPAN vol. 011, no. 107 (P-563), 4 April 1987 & JP-A-61 256249 (FUJITSU GENERAL LTD), 13 November 1986,

## Description

The present invention proposes a system and the related device for calibrating sensors for the measurement of the water contents on sheet, tape or film materials.

It can be applied, for example, to the paper production plants, where the need is felt to keep under control the water contents of the produced paper.

The use of moisture contents sensors on paper manufacturing plants is known.

The operation of said moisture meters is usually based on the evaluation, by means of suitable sensors, of the amount of electromagnetic radiation, in the range of infrared or of microwaves, absorbed by the material under measurement.

It is known that infrared and microwave radiation sensors exhibit a non-linear transfer function, and therefore, before actually using them, an operation is to.be carried out to calibrate them.

It is known that the moisture measurement carried out by means of infrared or microwave radiation sensors can be considerably influenced by the geometrical arrangement of the cellulose fibres.

It is known that the moisture measurement carried out by means of infrared sensors can be considerably influenced by the kind and amount of additives that have been employed in the preparation of the paper pulp.

The need of checking the accuracy in the moisture contents sensor calibration occurs therefore not only every time the production of a new paper quality is started, but also in the course of the manufacture thereof, in order to prevent apparently negligible changes in the paper characteristics from giving rise to intolerable errors in the moisture measurement of the produced paper web, thereby causing the quality of the finished product to be spoiled.

The calibration operation consists essentially in firstly, measuring the moisture of a set number of samples with different moisture contents of the paper to be produced with a standard instrument, followed by the sensor to be calibrated, and correcting thereafter the sensor reading to match the reading of the standard instrument.

WO 95/34076 (prior art under Article 54(3) EPC) discloses an apparatus for calibrating on line a moisture sensor comprising a calibration sensor which measures the same object measured by the measuring sensor and a communication link for compling the measuring sensor to the calibration sensor.

EP 0.453.797 discloses the use of paper samples with different moisture weight for achieving the calibration of a moisture meter.

JP 6229658 discloses an apparatus for calibrating a moisture sensor comprising a calibration container capable of production of standard humidity wherein the moisture sensor is periodically placed to recalibrate it.

The standard instrument tipically employed for determination of the water contents of the samples used for the calibration is a precision balance, and the water contents is calculated as the difference between the weight of the moist sample and the weight of the dry sample.

The higher the number of the samples used, the higher will obviously be the accuracy of the calibration curve. The calibration of the moisture contents sensor has been traditionally carried out manually because a possible automatization of the process would be complicated and expensive.

In fact, a possible manipulator ought to be able to:
- place and remove the sample onto and from a precision scale pan;
- introduce and remove the sample into and from a container, said container being transparent to be infrared radiation or the microwaves and tightly sealed in order to avoid the water contents of the sample to change as a consequence of the differences between the humidity and temperature of the surrounding atmosphere and the moisture and temperature of the sample.

The moisture contents sensors calibration operation, though simple in principle, requires therefore quite a long time to be carried out, tipically not less than three to four hours.

This turns out to be especially disadvantageous when the accuracy of the calibration curve is to be checked while the paper production plant is operating.

In fact, it is unconceivable to possibly stop temporarily the plant in order to carry out the moisture contents sensor calibration.

Yet, the long time required to carry out the calibration of a moisture contents sensor can cause a huge amount of material having an unsatisfactory quality to be produced.

The main object of the present is to provide a calibration system for moisture contents sensors for sheet, tape or film materials that would eliminate the need of using a tightly sealed container to put the calibration samples in.

Another object of the present is to provide a calibration system for moisture contents sensors for sheet, tape or film materials that would eliminate the need to put and remove the calibration samples onto and from a scale pan.

A further object of the present is to provide a calibration system for moisture contents sensors for sheet, tape or film materials that would allow the calibration operation to be carried out in a quick, satisfying manner.

A still further object of the present is to provide a calibration system for moisture contents sensors for sheet, tape or film materials that is suitable to be carried out automatically in a simple, cost-efficient manner.

A further important object of the present is to propose a calibration system for moisture contents sensors for sheet, tape or film materials in which the calibration curve can be determined in an automatic way.

These objects, as well as other objects that will be more clearly apparent from a reading of the following description, are achieved by means of a system for the calibration of moisture contents sensors for sheet, tape or film materials and by means of a related device according the characterizing part of the joined claims.

Further features and advantages of the invention will appear more clearly from the description of a preferred, non-limiting embodiment of the system and the related device for the calibration of an infrared or microwave radiation sensor to be used for the measurement of the moisture contents of sheet, tape or film materials, and of related devices that are shown in an illustrative, non-limiting way in the enclosed drawings, in which:
Figure 1 is a diagrammatic illustration of the system proposed according to a main embodiment;
Figure 2 is a diagrammatic illustration of a detail of the proposed system according to an alternative embodiment.

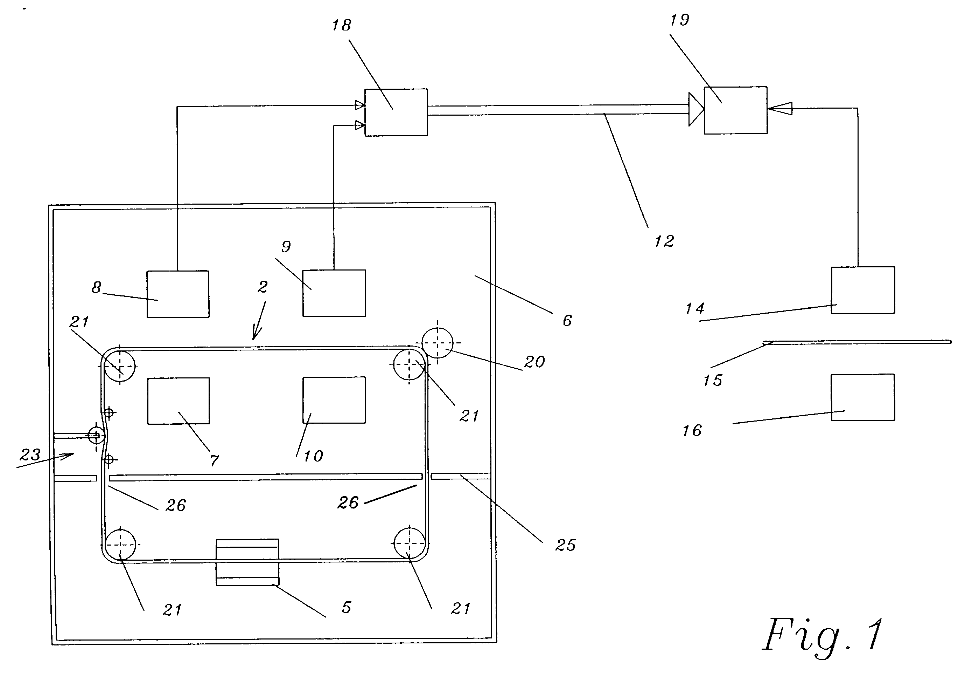

Referring to figure 1, numerals 14 and 16 designate a moisture meter of a production line and numeral 15 designates a paper sheet on which the water content is to be measured.

The moisture meter comprises a microwave or infrared radiation emitter 16 and a related sensor 14 the calibration of which is to be carried out.

A first important feature of the proposed invention is that the operation for defining the calibration curve is not carried out on sensor 14 on the production line, but on a second sensor 9 that is built exactly in the same manner as the first one, but located in a remote position.

The calibration curve as obtained by operating on said remote sensor 9 is then transmitted from a first computing unit 18 through a communication line 12 to a second computing unit 19 that monitors sensor 14 on the production line.

The characteristic of carrying out the calibration on a remote sensor allows the calibration system to be simply and cost-effectively arranged within a temperature and humidity controlled room 6, thereby eliminating the need of putting the paper samples into tightly closed containers as in the known art.

To explain it in greater detail, the calibration device according to the present invention comprises a paper sample 2, in the form of a web that is caused to advance by means of suitable pulling devices along a closed path on which a moisture meter consisting of an emitter 10 and a sensor 9 is provided, and of means 3 that are adapted to determine the weight of sample 2.

In particular, the pulling means comprise a plurality of idle wheels 21 which define the path that sample 2 is to follow, and a driving wheel 20 that presses onto sample 2 and that is arranged to the opposite side of one of the idle wheels 21.

A tensioning device 23 for keeping the paper sample 2 tense is also provided.

Also provided are means 5, such as an electric and/or microwave oven, to dry, if necessary, sample 2.

The oven 5 is separated from moisture meter 4 and from the means 3 that are adapted to press sample 2 by means of a wall 25 in which slits 26 are provided through which the sample web 2 runs.

A second feature of the invention is to determine the weight of the sample by means of a basis-weight sensor 7, 8 comprising a beta-radiation source 7 and an ionization chamber detector 8.

In this way, the sample can be weighed while simply running within the space comprised between source 7 and detector 8.

The operation of the system is as follows:
a sample 2 of the paper web that is being produced is driven by driving wheel 20.

The determination of the calibration curve is started from a moisture value near zero.

To this purpose, the humidity of room 6 is brought to the lowest possible value while, at the same time, switching on oven 5 in which the sample 2 is caused to circulate in order to dry it.

After that sample 2 has been dried and the humidity inside room 6 has reached the lowest possible value, the oven 5 is switched off and the basis-weight sensor carries out a measurement on the whole width of the sample, followed by calculating the average value of basis-weight and, consequently the average value of weight of sample 2.

The value of the dry weight is then stored in the computing unit 18 to be used later on as a reference value for the calculation of the water contents of moistened sample 2.

At the same time while the basis-weight of dry sample 2 is being measured, the infrared or microwave sensor 9 measures the average moisture of sample 2 and the value as measured by sensor 9 is finally corrected by putting it equal to zero.

The first point of the calibration curve has thus been defined.

A monotonically driven increase of humidity inside room 6 is then carried out until a preset number of desired humidity values are reached.

When each of the set humidity values is reached, the humidity inside room 6 remains constant for the time required in order to measure the average weight of the moist sample 2, calculate its water contents, measure its moisture by means of sensor 9 and carry out the correction of the value as measured by sensor 9, taking as a reference the value calculated by means of sensor 7 in order to define the related point of the calibration curve.

Once the calibration curve has been completed, it is transmitted from computing unit 18, through the communication line 12, to the computing unit 19 that monitors the line sensor 14.

The device thus provided allows therefore a fast and reliable calibration of moisture contents sensor 14 to be carried out without too high costs to be accepted or too heavy technical difficulties to be tackled.

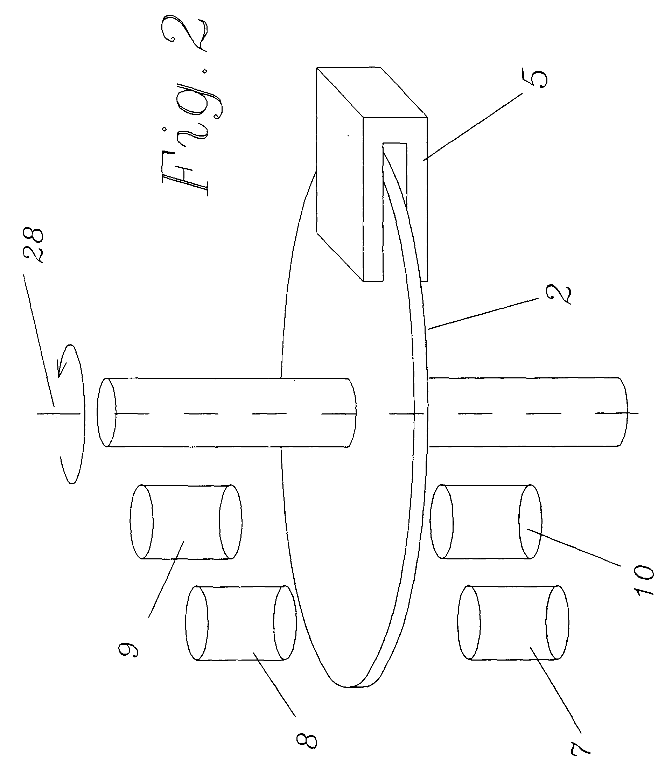

Figure 2 illustrates a detail of the proposed system in which the paper sample 2 instead of beeing a web driven along a closed circuit is a disk 2 rotating around its axis 28.

The basis-weight meter 7, 8 and the moisture meter 4 detect the average values on a circular crown portion of said disk 2.

Here too oven 5 is preferably screened by means of walls 25 that are suited to limit the effects of heating on meters 7, 8 and 9, 10 and the whole device is arranged in a room 6 with controlled temperature and moisture.

The invention as explained can be changed and modified in various way without departing from the scope of the inventive idea.

In practice the employed materials and dimensions can be modified according to the needs.

## Claims

1. A system for calibrating on-line a sensor (14) for the measurement of the moisture contents of sheet, tape or film materials comprising:
- a remote moisture meter (9, 10) kept inside a temperature and humidity controlled room (6), said moisture meter (9, 10) defining a measuring space for receiving a sample (2);
- a beta-ray basis weight meter (7, 8) kept inside said temperature and humidity controlled room (6), said beta-ray basis weight meter (7, 8) defining a measuring space for receiving a sample (2);
- a sample (2) moving along a path passing through said measuring spaces of said meters (9,10) and (7, 8);
- computing means (14) operatively connected to said meters (9, 10) and (7, 8) for determining the calibration curve of said remote moisture meter (9, 10); and
- transmission means (12) for transmitting the informations about said calibration curve

2. A device for calibrating a sensor (14) for the measurement of moisture contents of sheet, tape or film materials (15) characterized in that it comprises:
- a temperature and humidity-controlled room 6;
- a remote moisture meter (9, 10) inside said room (6) that is built in exactly the same way as the on-line meter (14, 16) to be calibrated;
- a beta-ray basis-weight meter (7, 8) inside said room (6);
- a drying oven (5) inside said room (6);
- a sample (2) arranged inside said room (6);
- insulating walls (25), in order to limit the influence of oven (5) onto meters (7, 8) e (9, 10), said walls being provided with slits (26) in order to allow the paper web 2 to pass through;
- driving means that are capable to drive the sample (2) along a closed path passing through said remote moisture meter (4), said basis-weight meter (3) and said drying oven (5);
- memory and computing means (18) for determining the calibration curve of the remote moisture contents sensor (9);
- means (12) to transfer the calibration curve to the computing unit (19) of the on-line moisture contents sensor (14).

3. A device as claimed in claim 2 characterized in that said sample 2 is a web.

4. A device as claimed in claim 2 and 3 characterized in that said driving means 1 comprise;
- at least one driving wheel (20);
- at least one idle wheel (21);
- at least one tensioning device (23).

5. A device is claimed in claim 2 characterized in that said sample 2 is a disk that rotates aound its axis.

6. A system according to claim 1 characterized in that said sheet, tape or film material is paper.

7. A device according to any of claims 2 to 5 characterized in that said sheed, tape or film material is paper.

## Patentansprüche

1. System zum Online-Eichen eines Sensors (14) zum Messen des Feuchtigkeitsgehalts von Blatt-, Band- oder Folienmaterialien, aufweisend:
- einen entfernt befindlichen Feuchtigkeitsmesser (9, 10), der innerhalb eines temperatur- und feuchtigkeitsgesteuerten Raumes (6) untergebracht ist, wobei der Feuchtigkeitsmesser (9, 10) einen Meßraum zum Empfangen eines Prüfkörpers (2) definiert;
- einen Betastrahlen-Basisgewichtsmesser (7, 8), der innerhalb des temperatur- und feuchtigkeitsgesteuerten Raumes (6) untergebracht ist, wobei der Betastrahlen-Basisgewichtsmesser (7, 8) einen Meßraum zum Empfangen eines Prüfkörpers (2) definiert;
- einen Prüfkörper (2), der sich entlang eines Weges bewegt, der durch die Meßräume der Messgeräte (9, 10) und (7, 8) verläuft;
- eine Berechnungseinrichtung (14), die bezüglich ihres Betriebs mit den Messgeräten (9, 10) und (7, 8) verbunden ist, um die Eichkurve des entfernt befindlichen Feuchtigkeitsmessers (9, 10) zu ermitteln; und
- eine Übertragungseinrichtung (12) zum Übertragen der Informationen über die Eichkurve.

2. Vorrichtung zum Eichen eines Sensors (14) zum Messen des Feuchtigkeitsgehalts von Blatt-, Band- oder Folienmaterialien (15), dadurch gekennzeichnet, daß sie folgendes aufweist:
- einen temperatur- und feuchtigkeitsgesteuerten Raum (6);
- einen entfernt befindlichen Feuchtigkeitsmesser (9, 10) innerhalb des Raums (6), der genau gleich aufgebaut ist wie das zu eichende Online-Messgerät (14, 16);
- einen Betastrahlen-Basisgewichtsmesser (7, 8) innerhalb des Raumes (6);
- einen Trockenofen (5) innerhalb des Raumes (6);
- einen Prüfkörper (2), der innerhalb des Raumes (6) angeordnet ist;
- Isolierwände (25), um den Einfluß des Ofens (5) auf die Messgeräte (7, 8) und (9, 10) zu begrenzen, wobei die Wände mit Schlitzen (26) versehen sind, um das Hindurchlaufen der Papierbahn (2) zu ermöglichen;
- eine Antriebseinrichtung, die in der Lage ist, den Prüfkörper (2) entlang eines geschlossenen Weges zu befördern, der durch den entfernt befindlichen Feuchtigkeitsmesser (4), den Basisgewichtsmesser (3) und den Trockenofen (5) hindurch verläuft;
- eine Speicher- und Berechnungseinrichtung (18) zum Ermitteln der Eichkurve des entfernt befindlichen Feuchtigkeitsgehaltssensors (9); und
- eine Einrichtung (12), um die Eichkurve an die Berechnungseinheit (19) des Online-Feuchtigkeitsgehaltssensors (14) zu übertragen.

3. Vorrichtung nach Anspruch 2, dadurch gekennzeichnet, daß der Prüfkörper (2) eine Gewebebahn ist.

4. Vorrichtung nach Anspruch 2 und 3, dadurch gekennzeichnet, daß die Antriebseinrichtung (1) folgendes aufweist:
- mindestens ein Antriebsrad (20);
- mindestens ein Leerlauf-Rad (21);
- mindestens eine Spannvorrichtung (23).

5. Vorrichtung nach Anspruch 2, dadurch gekennzeichnet, daß der Prüfkörper (2) eine Scheibe ist, die um ihre Achse rotiert.

6. System nach Anspruch 1, dadurch gekennzeichnet, daß das Blatt-, Band- oder Folienmaterial Papier ist.

7. Vorrichtung nach einem der Ansprüche 2 bis 5, dadurch gekennzeichnet, daß das Blatt-, Band- oder Folienmaterial Papier ist.

## Revendications

1. Système pour étalonner en ligne un détecteur (14) pour la mesure de la teneur en humidité de matériaux en feuille, en bande ou en film, comprenant :
- un appareil de mesure d'humidité à distance (9, 10) contenu à l'intérieur d'une enceinte (6) à température et humidité contrôlées, ledit appareil de mesure d'humidité (9, 10) définissant un espace de mesure pour recevoir un échantillon (2) ;
- un appareil de mesure du poids de base à rayons bêta (7, 8) contenu à l'intérieur de ladite enceinte (6) à température et humidité contrôlées, ledit appareil de mesure du poids de base à rayons bêta (7, 8) définissant un espace de mesure pour recevoir un échantillon (2) ;
- un échantillon (2) se déplaçant sur un trajet passant à travers lesdits espaces de mesure desdits appareils de mesure (9, 10) et (7, 8) ;
- des moyens de calcul (14) fonctionnellement raccordés auxdits appareils de mesure (9, 10) et 7, 8) pour déterminer la course d'étalonnage dudit appareil de mesure d'humidité à distance (9, 10) ; et
- des moyens de transmission (12) pour transmettre les informations relatives à ladite courbe d'étalonnage.

2. Dispositif pour étalonner un détecteur (14) pour la mesure de la teneur en humidité de matériaux en feuille, en bande ou en film (15), caractérisé en ce qu'il comprend :
- une enceinte à température et humidité contrôlées (6) ;
- un appareil de mesure d'humidité à distance (9, 10) à l'intérieur de ladite enceinte (6) qui est construit exactement de la même manière que l'appareil de mesure en ligne (14, 16) à étalonner ;
- un appareil de mesure du poids de base à rayons bêta (7, 8) à l'intérieur de ladite enceinte (6) ;
- un four de séchage (5) à l'intérieur de ladite enceinte (6) ;
- un échantillon (2) disposé à l'intérieur de ladite enceinte (6) ;
- des parois isolantes (25) destinées à limiter l'influence du four (5) sur les appareils de mesure (7, 8) et (9, 10), lesdites parois comportant des fentes (26) afin de laisser la bande de papier (2) traverser l'enceinte ;
- des moyens d'entraînement capables d'entraîner l'échantillon (2) le long d'un trajet fermé passant à travers ledit appareil de mesure d'humidité à distance (4), ledit appareil de mesure du poids de base (3) et ledit four de séchage (5) ;
- des moyens de mémoire et de calcul (18) pour déterminer la courbe d'étalonnage du détecteur (9) de la teneur en humidité à distance ;
- des moyens (12) pour transférer la courbe d'étalonnage à l'unité de calcul (19) du détecteur de la teneur en humidité en ligne (14).

3. Dispositif selon la revendication 2, caractérisé en ce que ledit échantillon (2) est une bande.

4. Dispositif selon la revendication 2 et la revendication 3, caractérisé en ce que les moyens d'entraînement (1) comprennent :
- au moins une roue d'entraînement (20) ;
- au moins une roue folle (21) ;
- au moins un dispositif de tension (23).

5. Dispositif selon la revendication 2, caractérisé en ce que l'échantillon (2) est un disque qui tourne autour de son axe.

6. Dispositif selon la revendication 1, caractérisé en ce que ledit matériau en feuille, en bande ou en film est du papier.

7. Dispositif selon l'une des revendications 2 à 5, caractérisé en ce que ledit matériau en feuille, en bande ou en film est du papier.
